(19) Europäisches Patentamt
European Patent Office
Office européen des brevets

(11) EP 1 028 952 B1

(12) EUROPEAN PATENT SPECIFICATION

(45) Date of publication and mention of the grant of the patent:
**31.07.2002 Bulletin 2002/31**

(21) Application number: **98956480.2**

(22) Date of filing: **03.11.1998**

(51) Int Cl.$^7$: **C07D 309/30**, C07C 45/60

(86) International application number:
**PCT/US98/23329**

(87) International publication number:
**WO 99/23088 (14.05.1999 Gazette 1999/19)**

# (54) VINYL ETHER DERIVATIVES

VINYLETHER DERIVATE

DERIVES D'ETHER VINYLIQUE

(84) Designated Contracting States:
**AT BE CH DE DK ES FI FR GB GR IE IT LI LU NL PT SE**

(30) Priority: **04.11.1997 US 64162 P**

(43) Date of publication of application:
**23.08.2000 Bulletin 2000/34**

(73) Proprietor: **UNION CARBIDE CHEMICALS & PLASTICS TECHNOLOGY CORPORATION**
**Danbury, Connecticut 06817-0001 (US)**

(72) Inventors:
- **ETZKORN, William, George**
  **Hurricane, WV 25526 (US)**
- **GALLEY, Richard, A.**
  **Belle Mead, NJ 08502 (US)**
- **RODBERG, Joan, Ayer**
  **Charleston, WV 25304 (US)**
- **PARISI, Paul, J.**
  **St. Lamber, Quebec J4P 2Y8 (CA)**

(74) Representative: **Hayes, Adrian Chetwynd et al**
**Boult Wade Tennant,**
**Verulam Gardens**
**70 Gray's Inn Road**
**London WC1X 8BT (GB)**

(56) References cited:
**DE-C- 977 093** **US-A- 2 546 018**

- **CURTIS W. SMITH ET AL.: "reactions of acrolein and related compounds.1.addition of vinyl ethers" JOURNAL OF THE AMERICAN CHEMICAL SOCIETY., vol. 73, November 1951, pages 5267-5270, XP002094064 DC US**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 028 952 B1

## Description

### Field of the Invention

The present invention generally relates to vinyl ether derivatives and more specifically relates to the manufacture of vinyl ether derivatives by the reaction of a vinyl alkyl ether and an α,β-unsaturated carbonylic compound in the presence of a lower alcohol.

### Background of the Invention

Reaction of vinyl alkyl ethers and α,β-unsaturated carbonylic compounds produce various derivatives of 3,4-dihydro-1,2-pyran. Derivatives of 3,4-dihydro-1,2-pyran, in turn, can be used to produce, among other things, glutaraldehyde. Glutaraldehyde is a highly reactive chemical compound which has found a variety of uses in the chemical and biological fields. Typical uses for glutaraldehyde include, for example, use as a biocide, a chemical intermediate for crosslinking protein and polyhydroxy compounds, and in the tanning of leathers. One method that has been proposed for the preparation of glutaraldehyde involves the synthesis of the ozonide of cyclopentene and the decomposition of this product to form among other products glutaraldehyde. Another method which has been proposed for the synthesis of glutaraldehyde comprises the treatment of its oxime with nitric oxide or with amyl nitrites. More commonly, glutaraldehyde can be prepared by the acidic hydrolysis of an alkoxydihydropyran, such as methoxydihydropyran (also referred to herein as "MDP"), such as described in U.S. Patent No. 2,546,018 issued March 20, 1951. Alkoxydihyropyrans can be manufactured for example, by the reaction of the α,β-unsaturated carbonylic compound and vinyl alkyl ether. Specifically, MDP can be manufactured by the reaction of acrolein and vinyl methyl ether (also referred to herein as "VME"). Such a process for MDP manufacture is described is U.S. Patent No. 2,514,168 issued July 4, 1950.

Vinyl alkyl ethers such as, for example, VME, can be manufactured in a number of different ways. For instance, vinyl alkyl ethers can be produced from a glycol ether by an indirect dehydration process which comprises converting a glycol ether into an ester-type intermediate and then decomposing the intermediate to obtain a vinyl ether. Vinyl alkyl ethers can also be prepared by subjecting an acetal to alcohol elimination such as disclosed in U.S. Patent No. 3,739,032 issued June 12, 1973, U.S. Patent No. 4,973,751 issued November 27, 1990, and U.S. Patent No. 5,130,435 issued July 14, 1992 or by subjecting a glycol ether to intermolecular dehydration in a gas phase in the presence of a catalyst to convert the glycol ether into an unsaturated ether directly in a one step reaction such as disclosed in U.S. Patent No. 5,650,544 issued July 22, 1997. A more common process for the manufacture of vinyl alkyl ethers employs an addition reaction between acetylene and alcohol using an alkaline catalyst, such as disclosed in U.S. Patent No. 1,959,927 issued May 22, 1934, U.S. Patent No. 2,472,084 issued June 7, 1949, U.S. Patent No. 3,358,041 issued December 12, 1967 and U.S. Patent No. 3,370,095 issued February 20, 1968.

Curtis W. Smith et al. in "Reactions of Acrolein and Related Compounds I. Addition of Vinyl Ethers", Journal of the American Chemical Society., vol. 73, November 1951, pages 5267-5270, disclose simple thermal addition of acrolein and vinyl ethers to give 2-alkoxy-dihydropyrans in high yield. The authors further disclose that divinyl ether, phenyl vinyl sulfide, methacrolein and crotonaldehyde also add to give 3,4-dihydro-2H-pyrans substituted in the 2- rather than the 3- position. Also, DE 977093, issued January 28, 1965, discloses the preparation of various vinyl ether derivatives.

Vinyl alkyl ethers, such as, for example, vinyl methyl ether, have a variety of chemical uses including for example, as copolymers used in coatings and laquers, as modifiers for alkyl, polystryrene and ionomer resins, plasticizers for nitrocellulose, adhesives and as an intermediate for lubricating oils, pesticides, or pharmaceuticals. Vinyl methyl ether can also be used as a raw material in the preparation of MDP by the reaction of vinyl methyl ether with acrolein. Typically, when vinyl methyl ether is used to manufacture MDP, the VME is supplied in a relatively highly purified condition, e.g., above 98 wt. % vinyl methyl ether. Typical impurities in the vinyl alkyl ether include alcohols, water, aldehydes, and dialkyl ethers, among others. Depending on the manufacturing process, considerable effort can be involved in producing a highly purified vinyl alkyl ether product. Distillation columns, wash columns, driers, evaporators and the like must be used either alone, or in combination, to produce the highly purified vinyl alkyl ether. Typically, the vinyl methyl ether is used in excess and in some cases as the solvent for the reaction with acrolein in order to maximize acrolein conversion. Typical conversions of acrolein in its reaction with vinyl methyl ether to produce MDP in such reactions range from 20 to 60 percent.

Accordingly, processes are desired for the manufacture of MDP and other vinyl ether derivatives based on the reaction of vinyl alkyl ethers and α,β-unsaturated carbonylic compounds which are integrated with processes for the manufacture of the vinyl alkyl ether and which do not require the use of highly purified vinyl alkyl ethers. It is further desired that such integrated processes would provide enhanced conversions of the reactants to the vinyl ether derivative, preferably without substantially changing the reaction conditions. It is further desired that the processes which subsequently use the vinyl ether derivative as an intermediate in production of a final product, such as for example

glutaraldehyde, can be integrated with the vinyl ether derivative and the vinyl alkyl ether production facility such that a purified vinyl ether derivative is not required for use in the process.

## Summary of the Invention

The present invention provides a process for the manufacture of a vinyl ether derivative, comprising:

(a) reacting acetylene with a lower alcohol, containing at least one hydroxyl group and having from 1 to 10 carbon atoms, in an acetylene reaction zone under reaction conditions effective to form a vinyl alkyl ether;
(b) withdrawing from the acetylene reaction zone an ether effluent stream comprising the vinyl alkyl ether, acetylene and the lower alcohol;
(c) passing at least a portion of the ether effluent stream comprising the vinyl alkyl ether and lower alcohol to a vinyl ether reaction zone wherein the vinyl alkyl ether is reacted with one or more α,β-unsaturated carbonylic compounds to form the vinyl ether derivative; and
(d) withdrawing from the vinyl ether reaction zone a derivative effluent stream comprising the vinyl ether derivative, the α,β-unsaturated carbonylic compound, the vinyl alkyl ether, and the lower alcohol and recycling a portion of the derivative effluent stream comprising the lower alcohol to the acetylene reaction zone,

The present invention further provides a process for the manufacture of a methoxydihydropyran comprising:

(a) reacting acetylene with methanol in an acetylene reaction zone under reaction conditions effective to form vinyl methyl ether;
(b) withdrawing from the acetylene reaction zone an ether effluent stream comprising the vinyl methyl ether, acetylene and methanol; and
(c) passing at least a portion of the ether effluent stream comprising the vinyl methyl ether and methanol to a vinyl ether reaction zone wherein the vinyl methyl ether is reacted with acrolein to form methoxydihydropyran; and
(d) withdrawing from the vinyl ether reaction zone a derivative effluent stream comprising the methoxydihydropyran, the acrolein, the vinyl methyl ether, and the methanol and recycling a portion of the derivative effluent stream comprising the methanol to the acetylene reaction zone.

By the present invention, improved processes for the manufacture of vinyl ether derivatives, e.g., MDP, are provided by the reaction of a vinyl alkyl ether, e.g., VME, and an unsaturated α,β-unsaturated carbonylic compound, e.g., acrolein, in the presence of a lower alcohol, e.g., methanol. Quite surprisingly in accordance with the present invention, it has been found that the presence of the lower alcohol in the reaction of the vinyl alkyl ether and the α,β-unsaturated carbonylic compound can enhance the conversion of the vinyl alkyl ether and the α,β-unsaturated carbonylic compound.

The manufacture in such an integrated facility would start with, for example, acetylene reacting with, for example, methanol to form VME. The subsequently produced VME could be used in an impure state and can contain some or all of the unreacted methanol. This crude VME could be used to react with, for example, acrolein to produce MDP. The subsequently produced MDP containing some or all of the methanol could be used to produce glutaraldehyde. The subsequently produced glutaraldehyde would then be refined to the desired purity for use as a final product. Such an integrated facility would refine the various intermediates as necessary to have recycle streams allowing passage of unreacted materials back into the process where they can be utilized but extreme measures would not be taken to obtain purified intermediates of the vinyl alkyl ether or the vinyl ether derivative.

## Detailed Description of the Invention

The acetylene suitable for use in accordance with the present invention is readily commercially available and usually provided by pipeline from an acetylene source, e.g., a partial oxidation facility, a hydrocarbon cracking facility, or a calcium carbide facility. The specific process utilized to manufacture the acetylene is not critical to the present invention. The purity of the acetylene feedstream is not critical but typically will comprise at least 98 mol percent acetylene, preferably at least 99 mol percent acetylene. Typical impurities in the acetylene feedstreams depend on the type of manufacturing process utilized but could include ethylene, acetone, pentanes, nitrogen, propadiene, higher acetylenes, oxygen, water and others.

At elevated temperatures and pressures, acetylene is unstable and due care must be exercised in handling acetylene in order to minimize the danger of explosions. In commercial practice it is common to dilute the acetylene with an inert gas such as, for example, nitrogen, methane, ethane, hydrogen, or mixtures of these in order to render the acetylene containing stream non-explosive under the operating conditions of the process. Further details concerning the acetylene suitable for use in accordance with the present invention and the safe handling of acetylene are known to those skilled in the art.

As used herein, the term "lower alcohols" means organic compounds containing at least one hydroxyl group and having from 1 to 10 carbon atoms per molecule. The alcohols may be chosen from volatile monohydric aliphatic and cyclic, i.e. hydroaromatic and aralkyl alcohols, and from volatile partially etherified or partially esterified polyhydric alcohols. Examples of these types include, alcohols, such as methanol, ethanol, propanol (n- and iso-), butanols, hexanols, octanol, decanol, benzyl alcohol, 1-phenyl-3-propanol, cyclohexanol, the mono-ethers, such as mono-methyl, -ethyl, -butyl and -phenyl ethers and the corresponding ethers of polyhydric alcohols containing one free hydroxyl group, such as the di-alkyl ethers of glycerol. Preferably the lower alcohol is selected from the group consisting of methanol, ethanol, propanol, butanol, and mixtures thereof.

In accordance with the present invention, the acetylene and the lower alcohol are reacted under reaction conditions effective to form a vinyl alkyl ether. The purity of the lower alcohol feedstream is not critical but typically will comprise at least 90 wt. % methanol, preferably at least 95 wt. % methanol. The specific process utilized to manufacture the vinyl alkyl ether is not critical to the present invention. For instance, the vinyl methyl ether can be prepared by reacting the acetylene and alcohol at a relatively high pressure, e.g., 0.7 to 2.8 MPa gauge (100 to 400 psig) under dilution with an inert gas such as nitrogen, methane, ethane, hydrogen, or mixtures of these at a temperature of between 100 to 200°C. This type of process is disclosed, for example, in U.S. Patent No. 1,959,927. In another process, the vinylation of the lower alcohol with acetylene is carried out at medium pressures, e.g., 0 to 0.42 MPa gauge (0 to 60 psig) and a temperature of between 100 to 200°C. The acetylene is rendered non-explosive by combining the acetylene with the vapors of a normally liquid, suitable diluent. Suitable compounds for use as diluents include lower boiling liquid hydrocarbons, ethers, esters, aromatic or naphthenic hydrocarbons and the like. Mixtures of inert diluents with reactants may also be employed. The acetylene, diluent, and alcohol are introduced into a reactor in the presence of a high boiling reaction medium and a catalyst such as an alkali-metal alkoxide. The reaction medium should have a boiling point higher than the reaction temperature such as di-n-butyl acetal, mineral oil, a higher alcohol, e.g., 10 to 22 carbon atoms, a vinyl ether of the higher alcohol, or mixtures of the alcohol and its vinyl ether. The reaction medium is in the form of a stationary phase to which is added the reactant lower alcohol and acetylene and from which the product vinyl ether of the lower alcohol, together with unconverted reactants, is removed. Further details of such a process are disclosed in, for example, U.S. Patent No. 2,472,084 issued June 7, 1949 and U. S. Patent No. 3,370,095 issued February 20, 1968.

Preferably, in accordance with the present invention, the acetylene is combined with the vapor of the lower alcohol instead of being diluted with an inert gas or the vapors of other normally liquid diluents. The acetylene partial pressure is increased for a given total pressure and the overall reaction rate at a given temperature and pressure is increased. The ratio of acetylene to lower alcohol may be varied over a considerable range, since unreacted material can be readily recovered and recycled. Preferably, the lower alcohol is employed in excess of the stoichiometric amount in order to minimize the amount of unreacted acetylene present. Lower alcohol to acetylene molar ratios are typically 1:1.2 to 10:1. Lower molar ratios can be employed but that necessitates the presence of another inert diluent to maintain a safe concentration of acetylene in the system.

Typically, in accordance with the present invention, the reaction is conducted at a temperature of from 100 to 200°C, preferably from 120 to 180°C and more preferably from 140 to 160°C, and a pressure from 0 to 0.7 MPa gauge (0 to 100 psig), preferably from 0.14 to 0.56 MPa gauge (20 to 80 psig) and more preferably from 0.21 to 0.42 MPa gauge (30 to 60 psig).

Preferably, the reaction is carried out in the presence of a strongly alkaline vinylation catalyst, such as an alkali-metal oxide or hydroxide, an alkali-metal cyanide or an alkali-metal alkoxide or aryl oxide. An especially preferred catalyst is an alkyl metal hydroxide, such as potassium hydroxide, or an alkyl metal alkoxide derived from the alcohol such as potassium methoxide. Typically, the concentration of catalyst is from 1 to 30 wt. %, preferably from 2 to 25 wt. % and more preferably from 5 to 20 wt. % based on the weight reacting medium.

The reaction can be run in a continuous or a batch manner. Preferably, the reaction is conducted in a continuous manner. The rate at which the reactants are added will vary depending upon equipment used, reaction conditions, the nature of the reactants, etc. Generally, an addition rate corresponding to between 0.05 and 5 moles of acetylene per hour per mole of catalyst will be satisfactory. As used herein, the term conversion means moles of reactant converted per mole of reactant fed per pass through the reaction system. For example if 100 mol of acetylene is fed into the reactor and 10 mol of acetylene remains unconverted in the reactor effluent, the conversion would be:

$$\frac{(100\ mol\ acetylene\ fed - 10\ mol\ acetylene\ unconverted)}{100\ mol\ acetylene\ fed} = 90\%\ aceetylene\ conversion$$

In the reaction of acetylene with the lower alcohol to produce the vinyl alkyl ether, the acetylene conversion is 25 to 100 %, preferably 50 to 100%, and more preferably 90 to 100 %. The effluent from the described reactor contains the

unreacted lower alcohol, unreacted acetylene, and product vinyl alkyl ether.

In accordance with the present invention the vinyl alkyl ether so produced with any remaining unreacted methanol can be passed on to subsequent reaction systems with minimal refining. The vinyl alkyl ether can be refined such that the effluent can contain 10 to 99 mole % vinyl alkyl ether, preferably 20 to 99 mol % vinyl alkyl ether, and more preferably 33 to 99 mol % vinyl alkyl ether. With the present invention, the number of refining steps would be reduced to simple distillation leaving the so produced vinyl alkyl ether with contained lower alcohol.

Further details or variations in the process of this invention of the reaction of acetylene and alcohols to form vinyl alkyl ethers will be apparent to those skilled in the art.

The vinyl alkyl ethers produced by the processes of the present invention include any vinyl ethers of the lower alcohols used in the reaction with acetylene. Preferably, the vinyl alkyl ether has a formula:

$$CH_2 = CH - OR_1$$

wherein $R_1$ is a substituted or unsubstituted aliphatic or aromatic hydrocarbon radical having from 1 to 10 carbon atoms. Preferably $R_1$ is selected from the group consisting of methyl, ethyl, propyl, butyl, or mixtures thereof. More preferably, the vinyl alkyl ether is vinyl methyl ether.

The α,β-unsaturated carbonylic compounds may be defined as compounds which contain the grouping of atoms represented in the formula shown below

$$HR_2C=CR_3 - C(=O) - R_4$$

where, $R_2$, $R_3$, and $R_4$ may be the same or different and are hydrogen or a substituted or unsubstituted aliphatic or aromatic hydrocarbon radical having from 1 to 8 carbon atoms. Such compounds, suitable for reacting with the vinyl alkyl ethers produced in accordance with the present invention, have from 3 to 11 carbon atoms per molecule. Preferably, the α,β-unsaturated carbonylic compounds are aldehydes. More preferably, the α,β-unsaturated carbonylic compounds are selected from the group consisting of acrolein, crotonaldehyde, methacrolein, α-ethyl acrolein, ethyl propyl acrolein, cinnamaldehyde, and mixtures thereof. Most preferably, the α,β-unsaturated carbonylic compound is acrolein wherein $R_2$, $R_3$, and $R_4$ are all hydrogen.

In accordance with the present invention the vinyl alkyl ether is reacted with the α,β-unsaturated carbonylic compound as defined above to produce a derivative of 3,4-dihydro-1,2-pyran. The purity of the α,β-unsaturated carbonylic compound feedstream is not critical but typically will comprise at least 30 wt. % α,β-unsaturated carbonylic compound, preferably at least 55 wt. % α,β-unsaturated carbonylic compound, and more preferably at least 90 wt % α,β-unsaturated carbonylic compound. The ratio of reactants typically ranges from 1 to 5 moles of the vinyl alkyl ether per mol of the α,β-unsaturated carbonylic compound.

In accordance with the present invention there is present in the reaction stream, unreacted lower alcohol from the vinyl alkyl ether production step described above. The vinyl alkyl ether reactor feedstream would contain 1 to 90 wt. % of the lower alcohol, preferably 1 to 50 wt. % of the lower alcohol, and more preferably 1 to 40 wt. % lower alcohol, said percentages based on the total weight of the vinyl alkyl ether, α,β-unsaturated carbonylic compound, and the lower alcohol. Preferably, the amount of lower alcohol is effective to enhance the conversion of the vinyl alkyl ether and the α,β-unsaturated carbonylic compound to the vinyl ether derivative. Typically, the vinyl alkyl ether is used in excess to maximize the conversion of the α,β-unsaturated carbonylic compound. Preferably in accordance with the present invention, the conversion of the α,β-unsaturated carbonylic compound to the vinyl ether derivative is at least 20 percent, more preferably at least 40 percent and most preferably at least 60 percent. As used herein, the term reaction rate means moles of reactants converted per unit time. Preferably the reaction rate will increase at least 10%, more preferably the reaction rate will increase at least 50%, and more preferably the reaction rate will increase at least 100% over the case where there is a substantial absence of the lower alcohol. As used herein, the term substantial absence means less than 5 wt. %, preferably less than 2 wt. % and more preferably less than 1 wt. % based on the total weight of the vinyl alkyl ether, α,β-unsaturated carbonylic compound and the lower alcohol. The presence of for example, methanol in the reaction mixture in the vinyl ether reaction zone accelerates the reaction of the vinyl methyl ether with acrolein to produce the methoxydihydropyran. Therefore, under a given set of reaction conditions of reactant concentration, reaction temperature, reaction pressure, and residence time, the presence of methanol results in higher conversions of the vinyl methyl ether and acrolein than is obtained under the same reaction conditions in the substantial absence of methanol.

The reaction conditions suitable for reacting the vinyl alkyl ether with the α,β-unsaturated carbonylic compound will vary depending upon the particular α,β-unsaturated carbonylic compound and lower alcohol employed. Preferably, however, the reaction is conducted in a continuous mode in the liquid phase. The temperature of the reaction is typically

from 100 to 200 °C, and preferably from 120 to 190°C, and more preferably from 140 to 180°C. The pressure is typically from 0.105 to 7 MPa gauge (15 to 1000 psig) and preferably from 3.5 to 5.95 MPa gauge (500 to 850 psig) and more preferably from 4.2 to 5.6 MPa gauge (600 to 800 psig). The reaction is typically conducted in the absence of a catalyst. The α,β-unsaturated carbonylic compound usually contains an inhibitor to minimize polymer formation. Typical inhibitors include, for example, hydroquinone, butylhydroxytoluene and phenolphthiazine. Appropriate use of the inhibitors is known to those skilled in the art. The reaction is typically conducted in the absence of added solvent, the excess vinyl alkyl ether acting as solvent for the α,β-unsaturated carbonylic compound and the vinyl ether derivative. An inert atmosphere is used to prevent undesired side reactions and to minimize the risk of developing an explosive vapor composition in the reaction equipment. Such a process is described in U.S. Patent 2,514,168. Further details concerning the reaction of vinyl alkyl ether or α,β-unsaturated carbonylic compounds are known to those skilled in the art.

The vinyl ether derivative so produced would be refined in the process such that any unreacted vinyl alkyl ether and α,β-unsaturated carbonylic compound would be recycled to a point in the process prior to the reactors which react the vinyl alkyl ether and the α,β-unsaturated carbonylic compounds.

In accordance with the present invention, the vinyl ether derivative, so produced, would contain unreacted lower alcohol from the VME manufacture step described above. The vinyl ether derivative stream would contain 1 to 90 wt. % of the lower alcohol, preferably 1 to 50 wt. % of the lower alcohol, and more preferably 1 to 25 wt. % lower alcohol, said percentages based on the total weight of the reactor effluent.

The vinyl ether derivatives produced by the processes of the present invention include any reaction products of the vinyl alkyl ethers and the α,β-unsaturated carbonylic compounds. Preferably, the vinyl ether derivatives have the formula:

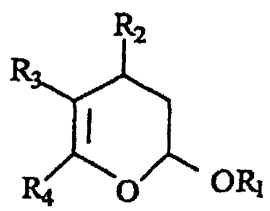

wherein $R_1$ is a substituted or unsubstituted aliphatic or aromatic hydrocarbon radical having from 1 to 10 carbon atoms, $R_2$, $R_3$ ,and $R_4$ may be the same or different and are hydrogen or a substituted or unsubstituted aliphatic or aromatic hydrocarbon radical having from 1 to 8 carbon atoms. More preferably, the vinyl ether derivative is an unsubstituted alkoxy dihydropyran and most preferably the vinyl ether derivative is methoxydihydropyran (MDP).

The vinyl ether derivatives produced by the processes of the present invention have a variety of end uses known to those skilled in the art, such as for example, use as copolymers and as intermediates in chemical reactions. When the vinyl ether derivative is MDP, one preferred use is as a reactant in the production of glutaraldehyde. Preferably in accordance with the present invention, the MDP is hydrolyzed under acidic conditions to form glutaraldehyde and methanol. This reaction is typically conducted at a temperature of from 50 to 150°C and preferably from 75 to 125°C and at a pressure of from 0 to 0.7 MPa gauge (0 to 100 psig), preferably from 0.035 to 0.35 MPa gauge (5 to 50 psig). Typically, the reaction is conducted in a liquid phase under stirring conditions. The acidic environment can be provided for example, by a dilute aqueous solution of a mineral acid or by an aqueous solution of an organic acid. Such a process is disclosed in U.S. Patent No. 2,546,018 issued March 20, 1951.

The lower alcohol produced in the reaction of the hydrolysis of the vinyl ether derivative is separated from the glutaraldehyde and can be recycled back at an appropriate position within the process. It is desired that the glutaraldehyde product contains 0.01 to 3 % of the lower alcohol, preferably 0.01 to 1.5 wt. % lower alcohol, and more preferably 0.01 to 1 wt. % lower alcohol.

In accordance with the present invention, the lower alcohol contained within the vinyl ether derivative stream can be carried into the hydrolysis reactor and subsequently separated with the lower alcohol co-product.

Other suitable processes for the preparation of glutaraldehyde include, for example the reaction of alkoxydihydropyrans with water in the presence of bleaching earths as catalysts such as disclosed in U.S. Patent No. 5,600,018. Further details concerning the manufacture of glutaraldehyde are known to those skilled in the art.

The glutaraldehyde produced by the processes of the present invention, has a variety of end uses, for example as a biocide, an intermediate for crosslinking protein and polyhydroxy compounds and for the tanning of leathers.

Further details concerning the process conditions, materials, handling and apparatus suitable for use in accordance with the present invention are known to those skilled in the art.

The invention is hereinafter described with reference to Figure 1 which is not intended to limit the scope of

the claims which follow.

A methanol feed stream comprising 99 mol % methanol is fed to the process via line 13 and is combined with an alcohol recycle stream 14 comprising 95 wt. % methanol, the source of which is hereinafter defined to form line 27 which is introduced to a alcohol vaporizer zone 107. The alcohol vaporizer zone 107 is operated at a temperature from 50 to 150°C and a pressure of 0 to 0.14 MPa gauge (0 to 20 psig).

The methanol vapor stream 28 is withdrawn from the alcohol vaporizer zone 107 and combined with an acetylene feed stream 10 which contains 99 mol % acetylene and an acetylene recycle stream 11 which contains 60 to 90 wt. % vinyl methyl ether with the balance being mainly acetylene, the source of which is hereinafter described, to form line 12 which is then compressed and introduced to an acetylene reaction zone 100. The feedstreams to acetylene reaction zone 100 are controlled to maintain a ratio of reactants of from 1 to 5 mols of methanol per mol of the acetylene. The temperature of the alcohol vaporizer zone 107 is controlled so that this desired mole ratio of methanol to acetylene is maintained in the vapor state (i.e. no condensation of methanol occurs) after mixing with acetylene feedstream 10 which may or may not be preheated prior to mixing with stream 28. It is also possible that the acetylene feed stream 10 be fed directly to the alcohol vaporizer, as may the acetylene recycle feed stream 11. The particular choice of mixing positions are numerous and can be determined by those skilled in the art.

Acetylene reaction zone 100 is comprised of a continuous stirred tank reactor or a packed tower reactor with a means of heat removal sufficient to remove the heat of reaction and a means of effecting efficient contact between the vapor reactants and the liquid phase alkaline catalyst. The reactor is operated at a temperature from 140 to 160°C and a pressure of 0.21 to 0.42 MPa gauge (30 to 60 psig).

An ether effluent stream 16 comprising acetylene, methanol, vinyl methyl ether, and other minor impurities is withdrawn from acetylene reaction zone 100 and passed to an acetylene separation zone 101 which is comprised of a distillation column having from 3 to 20 stages. Variations in the distillation column heating or cooling will dictate the degree of separation between the ether and the alcohol. Acetylene separation zone 101 is operated with a column base temperature of from 40 to 60°C and a column pressure of 0 to 0.14 MPa gauge (0 to 20 psig). An acetylene recycle stream 11 comprising 60 to 90 wt. % VME is withdrawn as an overhead stream from the acetylene separation zone 101 and passed to acetylene reaction zone 100 as described above. The composition of acetylene recycle stream 11 can be varied by changing the overhead condenser cooling in the acetylene separation zone. A heavies methanol stream 15 comprising 95 wt.% methanol and 5 wt. % heavies is withdrawn as a bottom stream from acetylene separation zone 101 and passed to refining column 104 described hereinafter. An intermediate ether stream 17 is withdrawn as a side cut from acetylene separation zone 101 and comprises from 50 to 99 wt. % VME and from 1 to 50 wt. % methanol.

Intermediate ether stream 17 is combined with a crude or refined acrolein feedstream 19, and a light derivative stream 18 comprising unreacted acrolein, VME and methanol, the source of which is described hereinafter, to form stream 30. Stream 30 is fed through a high pressure pump capable of compressing the liquid feed stream to the desired operating pressure and subsequently introduced to a vinyl ether reaction zone 102 which comprises high pressure reactor with heat removal capabilities. The vinyl ether reaction zone 102 is operated at a temperature of from 140 to 180°C and a pressure of 4.2 to 5.6 MPa gauge (600 to 800 psig). The feedstreams are controlled to provide a reactant ratio of from 1 to 5 mols of VME per mol of acrolein and a concentration of methanol of 1 to 40 wt. %. The conversion of acrolein to MDP in vinyl ether reaction zone 102 is at least 60 %.

A derivative effluent stream comprising acrolein, VME, MDP and methanol is withdrawn from vinyl ether reaction zone 102 via line 20 and passed to a derivative separation zone 103. Derivative separation zone 103 is comprised of a distillation column with 10 to 40 stages. The derivative separation zone 103 is operated with a column base temperature of from 50 to 200°C and a pressure of from 0 to 0.7 MPa gauge (0 to 100 psig). Light derivative stream 18 comprising unreacted acrolein, VME and methanol is withdrawn from derivative separation zone 103 and recycled back to the vinyl ether reaction zone 102 as described above. A heavy derivative stream 21 comprising MDP, methanol and water is withdrawn from derivative separation zone 103 and introduced to a derivative refining zone 104 along with the heavies methanol stream 15 comprising methanol and vinyl methyl ether described above. Derivative refining zone 104 comprises a vacuum distillation column having 5 to 20 stages. The derivative refining zone 104 is operated with a column base temperature of from 75 to 150°C and a column pressure of 0.007 to 0.049 MPa absolute (1 to 7 psia). A refining bottoms stream 22 comprising undesired byproducts and other heavy components is withdrawn from derivative refining zone 104 and removed from the process.

A light refining stream 23 comprising MDP, methanol, and water is withdrawn as an overhead from derivative refining zone 104 and passed to a aldehyde reaction zone 105. Aldehyde reaction zone 105 comprises a multistage continuous stirred reactor. The aldehyde reaction zone 105 is operated at a temperature of from 75 to 125°C and a pressure of from 0.035 to 0.35 MPa gauge (5 to 50 psig). Acidified water is introduced to the aldehyde reaction zone via line 24. An aldehyde reaction effluent stream 25 comprising glutaraldehyde, methanol, water and MDP is withdrawn from the aldehyde reaction zone 105 and introduced to a aldehyde refining zone 106. Aldehyde refining zone 106 comprises a vacuum distillation column with 10-40 stages and neutralization of the product. The aldehyde refining zone is operated at a column base temperature of from 75 to 120°C and a pressure of from 0.021 to 0.105 MPa absolute

(3 to 15 psia). It is possible in the aldehyde refining zone to introduce acidic water above the inlet position of stream 25 such that any unreacted MDP traveling up the column can continue to react in refining zone 106 to product glutaraldehyde. In this case, the temperature of the refining zone would be elevated to allow for the reaction to proceed. It is also possible that the residence time within the refining zone would need to be minimized, for example with a low volume kettle or with wiped film evaporator, or the like. In another embodiement, the aldehyde reaction zone 105 and the refining zone 106 could be combined into a single zone where both reaction and refining occurred simultaneously. Alcohol recycle stream 14 comprising 95 wt. % methanol is withdrawn as an overhead stream from aldehyde refining zone 106 and introduced to acetylene reaction zone 100 as described above. A glutaraldehyde product stream 26 comprising glutaraldehyde is withdrawn from the aldehyde refining zone 106 and removed from the process.

## Claims

**1.** A process for the manufacture of a vinyl ether derivative, comprising:

(a) reacting acetylene with a lower alcohol, containing at least one hydroxyl group and having from 1 to 10 carbon atoms, in an acetylene reaction zone under reaction conditions effective to form a vinyl alkyl ether;
(b) withdrawing from the acetylene reaction zone an ether effluent stream comprising the vinyl alkyl ether, acetylene and the lower alcohol;
(c) passing at least a portion of the ether effluent stream comprising the vinyl alkyl ether and lower alcohol to a vinyl ether reaction zone wherein the vinyl alkyl ether is reacted with one or more α,β-unsaturated carbonylic compounds to form the vinyl ether derivative; and
(d) withdrawing from the vinyl ether reaction zone a derivative effluent stream comprising the vinyl ether derivative, the α,β-unsaturated carbonylic compound, the vinyl alkyl ether, and the lower alcohol and recycling a portion of the derivative effluent stream comprising the lower alcohol to the acetylene reaction zone.

**2.** The process of claim 1 further comprising recycling another portion of the derivative effluent stream comprising the lower alcohol, the vinyl alkyl ether, and the α,β-unsaturated carbonylic compound to the vinyl ether reaction zone.

**3.** The process of claim 1 or 2 wherein the vinyl alkyl ether is reacted with said one or more α,β-unsaturated carbonylic compounds in the presence of at least 1 wt. percent of the lower alcohol, said percentage based on the total weight of the vinyl alkyl ether, α,β-unsaturated carbonylic compounds and lower alcohol.

**4.** The process of claim 3 wherein the amount of the lower alcohol in the vinyl ether reaction zone is from 10 to 50 wt, % based on the total weight of the vinyl alkyl ether, α,β-unsaturated carbonylic compounds and lower alcohol.

**5.** The process of claim 4 wherein the amount of the lower alcohol in the vinyl ether reaction zone is from. 20 to 40 wt. percent based on the total weight of the vinyl alkyl ether, α,β-unsaturated carbonylic compounds and lower alcohol.

**6.** The process of any one of the preceding claims wherein the conversion of the α,β- unsaturated carbonylic compounds to the vinyl ether derivative in the vinyl ether reaction zone is at least 20 %.

**7.** The process of claim any one of the preceding claims wherein the lower alcohol is selected from the group consisting of methanol, ethanol, propanol, butanol and mixtures thereof.

**8.** The process of any one of the preceding claims wherein the α,β-unsaturated carbonylic compound has from 3 to 11 carbon atoms per molecule.

**9.** The process of claim 8 wherein the α,β-unsaturated carbonylic compound is an aldehyde or a ketone.

**10.** The process of claim 9 wherein the α,β-unsaturated carbonylic compound is selected from the group consisting of acrolein, crotonaldehyde, methacrolein, α-ethyl acrolein, ethyl propyl acrolein, and mixtures thereof.

**11.** The process of any one of the preceding claims wherein the vinyl alkyl ether has the formula:

$$CH_2 = CH - OR_1$$

wherein $R_1$ is a substituted or unsubstituted aliphatic or aromatic hydrocarbon radical having from 1 to 10 carbon atoms.

**12.** The process of claim 11 wherein $R_1$ is selected from the group consisting of methyl, ethyl, propyl, butyl or mixtures thereof.

**13.** The process of claim 12 wherein the vinyl alkyl ether is vinyl methyl ether.

**14.** The process of any one of the preceding claims wherein the vinyl ether derivative has the formula:

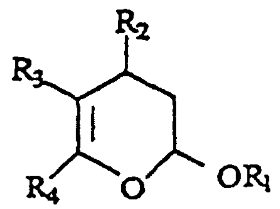

wherein $R_1$ is a substituted or unsubstituted aliphatic or aromatic hydrocarbon radical having from 1 to 10 carbon atoms, $R_2$, $R_3$, and $R_4$ may be the same or different and are hydrogen or a substituted or unsubstituted aliphatic or aromatic hydrocarbon radical having from 1 to ,8 carbon atoms.

**15.** The process of claim 14 wherein $R_2$, $R_3$, and $R_4$ are hydrogen.

**16.** The process of claim 15 wherein the vinyl ether derivative is methoxydihydropyran.

**17.** The process of claim 16 further comprising hydrolyzing the methoxydihydropyran to form glutaraldehyde and methanol.

**18.** The process of claim 17 further comprising recycling at least a portion of the methanol formed in the hydrolyzation of the methoxydihydropyran to the acetylene reaction zone.

**19.** A process for the manufacture of a methoxydihydropyran, comprising:

(a) reacting acetylene with methanol in an acetylene reaction zone under reaction conditions effective to form vinyl methyl ether;
(b) withdrawing from the acetylene reaction zone an ether effluent stream comprising the vinyl methyl ether, acetylene and methanol; and
(c) passing at least a portion of the ether effluent stream comprising the vinyl methyl ether and methanol to a vinyl ether reaction zone wherein the vinyl methyl ether is reacted with acrolein to form methoxydihydropyran; and
(d) withdrawing from the vinyl ether reaction zone a derivative effluent stream comprising the methoxydihydropyran, the acrolein, the vinyl methyl ether, and the methanol and recycling a portion of the derivative effluent stream comprising the methanol to the acetylene reaction zone.

**20.** The process of claim 19 further comprising withdrawing from the vinyl ether reaction zone a derivative effluent stream comprising the methoxydihydropyran, acrolein, vinyl methyl ether and methanol and passing a portion of the derivative effluent stream comprising methoxydihydropyran and methanol to an aldehyde reaction zone wherein the vinyl ether derivative is reacted with water under acidic reaction conditions to form glutaraldehyde and methanol.

**21.** The process of claim 20 further comprising recycling at least a portion of the methanol formed in the aldehyde reaction zone to the acetylene reaction zone.

## Patentansprüche

**1.** Verfahren für die Herstellung eines Vinyletherderivates, umfassend:

(a) Umsetzen von Acetylen mit einem niederen Alkohol, der wenigstens eine Hydroxylgruppe enthält und von 1 bis 10 Kohlenstoffatome hat, in einer Acetylenreaktionszone unter Reaktionsbedingungen, die zur Bildung eines Vinylalkylethers wirksam sind;
(b) Abziehen einer Etheraustrittsströmung, welche den Vinylalkylether, Acetylen und den niederen Alkohol umfaßt, aus der Acetylenreaktionszone;
(c) Führen von wenigstens einem Teil der Etheraustrittsströmung, welche den Vinylalkylether und den niederen Alkohol umfaßt, zu einer Vinyletherreaktionszone, worin der Vinylalkylether mit einer oder mehreren α,β-ungesättigten Carbonylverbindung/en zur Bildung des Vinyletherderivates umgesetzt wird; und
(d) Abziehen einer Derivataustrittsströmung, welche das Vinyletherderivat, die α,β-ungesättigte Carbonylverbindung, den Vinylalkylether und den niederen Alkohol umfaßt, aus der Vinyletherreaktionszone und Rückführen eines Teiles der Derivataustrittsströmung, welche den niederen Alkohol umfaßt, zu der Acetylenreaktionszone.

**2.** Verfahren von Anspruch 1, weiter umfassend das Rückführen eines anderen Teiles der Derivataustrittsströmung, welche den niederen Alkohol, den Vinylalkylether und die α,β-ungesättigte Carbonylverbindung umfaßt, zu der Vinyletherreaktionszone.

**3.** Verfahren von Anspruch 1 oder 2, bei welchem der Vinylalkylether mit dieser einen oder diesen mehreren α,β-ungesättigten Carbonylverbindung/en in Anwesenheit von wenigstens 1 Gew.-% des niederen Alkohols umgesetzt wird, wobei dieser Prozentsatz auf das Gesamtgewicht des Vinylalkylethers, der α,β-ungesättigten Carbonylverbindungen und des niederen Alkohols bezogen ist.

**4.** Verfahren nach Anspruch 3, bei welchem die Menge des niederen Alkohols in der Vinyletherreaktionszone von 10 bis 50 Gew.-% beträgt, bezogen auf das Gesamtgewicht des Vinylalkylethers, der α,β-ungesättigten Carbonylverbindungen und des niederen Alkohols.

**5.** Verfahren nach Anspruch 4, bei welchem die Menge des niederen Alkohols in der Vinyletherreaktionszone von 20 bis 40 Gew.-% beträgt, bezogen auf das Gesamtgewicht des Vinylalkylethers, der α,β-ungesättigten Carbonylverbindungen und des niederen Alkohols.

**6.** Verfahren nach einem der vorhergehenden Ansprüche, bei welchem die Umwandlung der α,β-ungesättigten Carbonylverbindungen in das Vinyletherderivat in der Vinyletherreaktionszone wenigstens 20 % beträgt.

**7.** Verfahren nach einem der vorhergehenden Ansprüche, bei welchem der niedere Alkohol ausgewählt ist aus der aus Methanol, Ethanol, Propanol, Butanol und Mischungen hiervon bestehenden Gruppe.

**8.** Verfahren nach einem der vorhergehenden Ansprüche, bei welchem die α,β-ungesättigte Carbonylverbindung von 3 bis 13 Kohlenstoffatome pro Molekül hat.

**9.** Verfahren nach Anspruch 8, bei welchem die α,β-ungesättigte Carbonylverbindung ein Aldehyd oder ein Keton ist.

**10.** Verfahren nach Anspruch 9, bei welchem die α,β-ungesättigte Carbonylverbindung aus der aus Acrolein, Crotonaldehyd, Methacrolein, α-Ethylacrolein, Ethylpropylacrolein und Mischungen hiervon bestehenden Gruppe ausgewählt ist.

**11.** Verfahren nach einem der vorhergehenden Ansprüche, bei welchem der Vinylalkylether die Formel hat:

$$CH_2=CH\text{-}OR_1$$

worin $R_1$ ein substituierter oder nicht-substiuierter, aliphatischer oder aromatischer Kohlenwasserstoffrest ist, der von 1 bis 10 Kohlenstoffatome besitzt.

**12.** Verfahren nach Anspruch 11, bei welchem $R_1$ aus der aus Methyl, Ethyl, Propyl, Butyl oder Mischungen hiervon

bestehenden Gruppe ausgewählt ist.

**13.** Verfahren nach Anspruch 12, bei welchem der Vinylalkylether Vinylmethylether ist.

**14.** Verfahren nach einem der vorhergehenden Ansprüche, bei welchem das Vinyletherderivat die Formel besitzt:

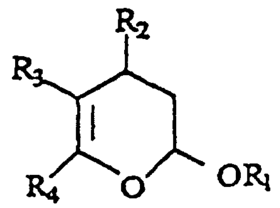

worin $R_1$ ein substituierten oder nicht-substiuierter, aliphatischer oder aromatischer Kohlenwasserstoffrest, der von 1 bis 10 Kohlenstoffatome besitzt, ist, $R_2$, $R_3$ und $R_4$ gleich oder verschieden sein können und Wasserstoff oder ein substituierter oder nicht-substiuierter, aliphatischer oder aromatischer Kohlenwasserstoffrest sind, der von 1 bis 8 Kohlenstoffatome besitzt.

**15.** Verfahren nach Anspruch 14, bei welchem $R_2$, $R_3$ und $R_4$ Wasserstoff sind.

**16.** Verfahren nach Anspruch 15, bei welchem das Vinyletherderivat Methoxydihydropyran ist.

**17.** Verfahren nach Anspruch 16, weiter umfassend die Hydrolyse des Methoxydihydropyrans zur Bildung von Glutaraldehyd und Methanol.

**18.** Verfahren nach Anspruch 17, weiter umfassend das Rückführen wenigstens eines Teiles des bei der Hydrolyse des Methoxydihydropyrans gebildeten Methanols zu der Acetylenreaktionszone.

**19.** Verfahren für die Herstellung eines Methoxydihydropyrans, umfassend:

(a) Umsetzen von Acetylen mit Methanol in einer Acetylenreaktionszone unter Reaktionsbedingungen, die zur Bildung von Vinylmethylether wirksam sind;
(b) Abziehen einer Etheraustrittsströmung, welche den Vinylmethylether, Acetylen und Methanol umfaßt, aus der Acetylenreaktionszone; und
(c) Führen von wenigstens einem Teil der Etheraustrittsströmung, welche den Vinylmethylether und Methanol umfaßt, zu einer Vinyletherreaktionszone, worin der Vinylmethylether mit Acrolein zur Bildung von Methoxydihydropyran umgesetzt wird; und
(d) Abziehen einer Derivataustrittsströmung, welche das Methoxydihydropyran, das Acrolein und das Methanol umfaßt, aus der Vinyletherreaktionszone und Rückführen eines Teiles der Derivataustrittsströmung, welche das Methanol umfaßt, zu der Acetylenreaktionszone.

**20.** Verfahren nach Anspruch 19, weiter umfassend das Abziehen einer Derivataustrittsströmung, welche das Methoxydihydropyran, Acrolein, Vinylmethylether und Methanol umfaßt, aus der Vinyletherreaktionszone und Führen eines Teiles der Derivataustrittsser unter sauren Reaktionsbedingungen zur Bildung von Glutaraldehyd und Methanol umgesetzt wird.

**21.** Verfahren nach Anspruch 20, weiter umfassend das Rückführen wenigstens eines Teiles des in der Aldehydreaktionszone gebildeten Methanols zu der Acetylenreaktionszone.

## Revendications

**1.** Procédé pour fabriquer un dérivé d'éther vinylique, comprenant :

(a) le fait de faire réagir l'acétylène avec un alcool inférieur comportant au moins un groupe hydroxyle et de 1 à 10 atomes de carbone, dans une zone de réaction de l'acétylène, dans des conditions réactionnelles

efficaces pour former un alkyléther vinylique ;
(b) le fait d'extraire de la zone de réaction de l'acétylène un courant d'effluent d'éther comprenant l'alkyléther vinylique, de l'acétylène et de l'alcool inférieur ;
(c) le fait de faire passer au moins une partie du courant d'effluent d'éther comprenant l'alkyléther vinylique et de l'alcool inférieur dans une zone de réaction de l'éther vinylique dans laquelle on fait réagir l'alkyléther vinylique avec un ou plusieurs composés carbonylés $\alpha,\beta$-insaturés pour former un dérivé d'éther vinylique ; et
(d) le fait d'extraire de la zone de réaction de l'éther vinylique un courant d'effluent de dérivé comprenant le dérivé d'éther vinylique, du composé carbonylé $\alpha,\beta$-insaturé, de l'alkyléther vinylique et de l'alcool inférieur, et le fait de recycler une partie du courant d'effluent de dérivé comprenant de l'alcool inférieur dans la zone de réaction de l'acétylène.

**2.** Procédé selon la revendication 1, comprenant en outre le fait de recycler une autre partie du courant d'effluent de dérivé comprenant de l'alcool inférieur, l'alkyléther vinylique et le composé carbonylé a,$\beta$-insaturé, dans la zone de réaction de l'éther vinylique.

**3.** Procédé selon la revendication 1 ou 2, dans lequel on fait réagir l'alkyléther vinylique avec ledit composé carbonylé $\alpha,\beta$-insaturé, au nombre d'au moins un, en présence d'au moins 1 % en poids d'alcool inférieur, ledit pourcentage étant rapporté au poids total d'alkyléther vinylique, de composés carbonylés $\alpha,\beta$-insaturés et d'alcool inférieur.

**4.** Procédé selon la revendication 3, dans lequel la quantité d'alcool inférieur dans la zone de réaction de l'éther vinylique est comprise entre 10 et 50 % en poids par rapport au poids total d'alkyléther vinylique, de composés carbonylés $\alpha,\beta$-insaturés et d'alcool inférieur.

**5.** Procédé selon la revendication 4, dans lequel la quantité d'alcool inférieur dans la zone de réaction de l'éther vinylique est comprise entre 20 et 40 % en poids par rapport au poids total d'alkyléther vinylique, de composés carbonylés $\alpha,\beta$-insaturés et d'alcool inférieur.

**6.** Procédé selon l'une quelconque des revendications précédentes, dans lequel le taux de conversion des composés carbonylés $\alpha,\beta$-insaturés en dérivé d'éther vinylique dans la zone de réaction de l'éther vinylique vaut au moins 20 %.

**7.** Procédé selon l'une quelconque des revendications précédentes, dans lequel l'alcool inférieur est choisi dans l'ensemble constitué par le méthanol, l'éthanol, le propanol, le butanol et leurs mélanges.

**8.** Procédé selon l'une quelconque des revendications précédentes, dans lequel le composé carbonylé $\alpha,\beta$-insaturé comporte de 3 à 11 atomes de carbone par molécule.

**9.** Procédé selon la revendication 8, dans lequel le composé carbonylé $\alpha,\beta$-insaturé est un aldéhyde ou une cétone.

**10.** Procédé selon la revendication 9, dans lequel le composé carbonylé $\alpha,\beta$-insaturé est choisi dans l'ensemble constitué par l'acroléine, le crotonaldéhyde, la méthacroléine, l'$\alpha$-éthylacroléine, l'éthylpropylacroléine, et leurs mélanges.

**11.** Procédé selon l'une quelconque des revendications précédentes, dans lequel l'alkyléther vinylique répond à la formule:

$$CH_2=CH-OR_1$$

dans laquelle $R_1$ représente un groupe hydrocarboné, aliphatique ou aromatique, substitué ou non, comportant de 1 à 10 atomes de carbone.

**12.** Procédé selon la revendication 11, dans lequel $R_1$ est choisi dans l'ensemble constitué par les groupes méthyle, éthyle, propyle, butyle et leurs mélanges.

**13.** Procédé selon la revendication 12, dans lequel l'alkyléther vinylique est le méthyléther vinylique.

**14.** Procédé selon l'une quelconque des revendications précédentes, dans lequel le dérivé d'éther vinylique répond

à la formule :

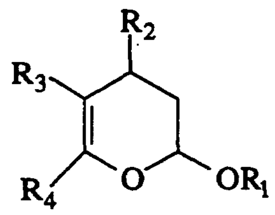

dans laquelle $R_1$ représente un groupe hydrocarboné, aliphatique ou aromatique, substitué ou non, comportant de 1 à 10 atomes de carbone, $R_2$, $R_3$ et $R_4$, identiques ou différents, représentent un atome d'hydrogène ou un groupe hydrocarboné, aliphatique ou aromatique, substitué ou non, comportant de 1 à 8 atomes de carbone.

**15.** Procédé selon la revendication 14, dans lequel $R_2$, $R_3$ et $R_4$, représentent des atomes d'hydrogène.

**16.** Procédé selon la revendication 15, dans lequel le dérivé d'éther vinylique est le méthoxydihydropyrane.

**17.** Procédé selon la revendication 16, comprenant en outre l'hydrolyse du méthoxydihydropyrane pour former le glutaraldéhyde et du méthanol.

**18.** Procédé selon la revendication 17, comprenant en outre le recyclage d'au moins une partie du méthanol formé au cours de l'hydrolyse du méthoxydihydropyrane, dans la zone de réaction de l'acétylène.

**19.** Procédé pour fabriquer le méthoxydihydropyrane, comprenant:

(a) le fait de faire réagir l'acétylène avec du méthanol, dans une zone de réaction de l'acétylène, dans des conditions réactionnelles efficaces pour former le méthyléther vinylique ;
(b) le fait d'extraire de la zone de réaction de l'acétylène un courant d'effluent d'éther comprenant le méthyléther vinylique, de l'acétylène et du méthanol ;
(c) le fait de faire passer au moins une partie du courant d'effluent d'éther comprenant le méthyléther vinylique et du méthanol dans une zone de réaction de l'éther vinylique dans laquelle on fait réagir le méthyléther vinylique avec l'acroléine pour former le méthoxydihydropyrane ; et
(d) le fait d'extraire de la zone de réaction de l'éther vinylique un courant d'effluent de dérivé comprenant le méthoxydihydropyrane, de l'acroléine, du méthyléther vinylique et du méthanol, et le fait de recycler une partie du courant d'effluent de dérivé comprenant du méthanol dans la zone de réaction de l'acétylène.

**20.** Procédé selon la revendication 19, comprenant en outre le fait d'extraire de la zone de réaction de l'éther vinylique, un courant d'effluent de dérivé comprenant le méthoxydihydropyrane, de l'acroléine, du méthyléther vinylique et du méthanol; et le fait de faire passer une partie du courant d'effluent de dérivé comprenant le méthoxydihydropyrane et du méthanol dans une zone de réaction d'un aldéhyde dans laquelle on fait réagir le dérivé d'éther vinylique avec de l'eau dans des conditions réactionnelles acides pour former le glutaraldéhyde et du méthanol.

**21.** Procédé selon la revendication 20, comprenant en outre le fait de recycler au moins une partie du méthanol formé dans la zone de réaction de l'aldéhyde, dans la zone de réaction de l'acétylène.

FIG. 1

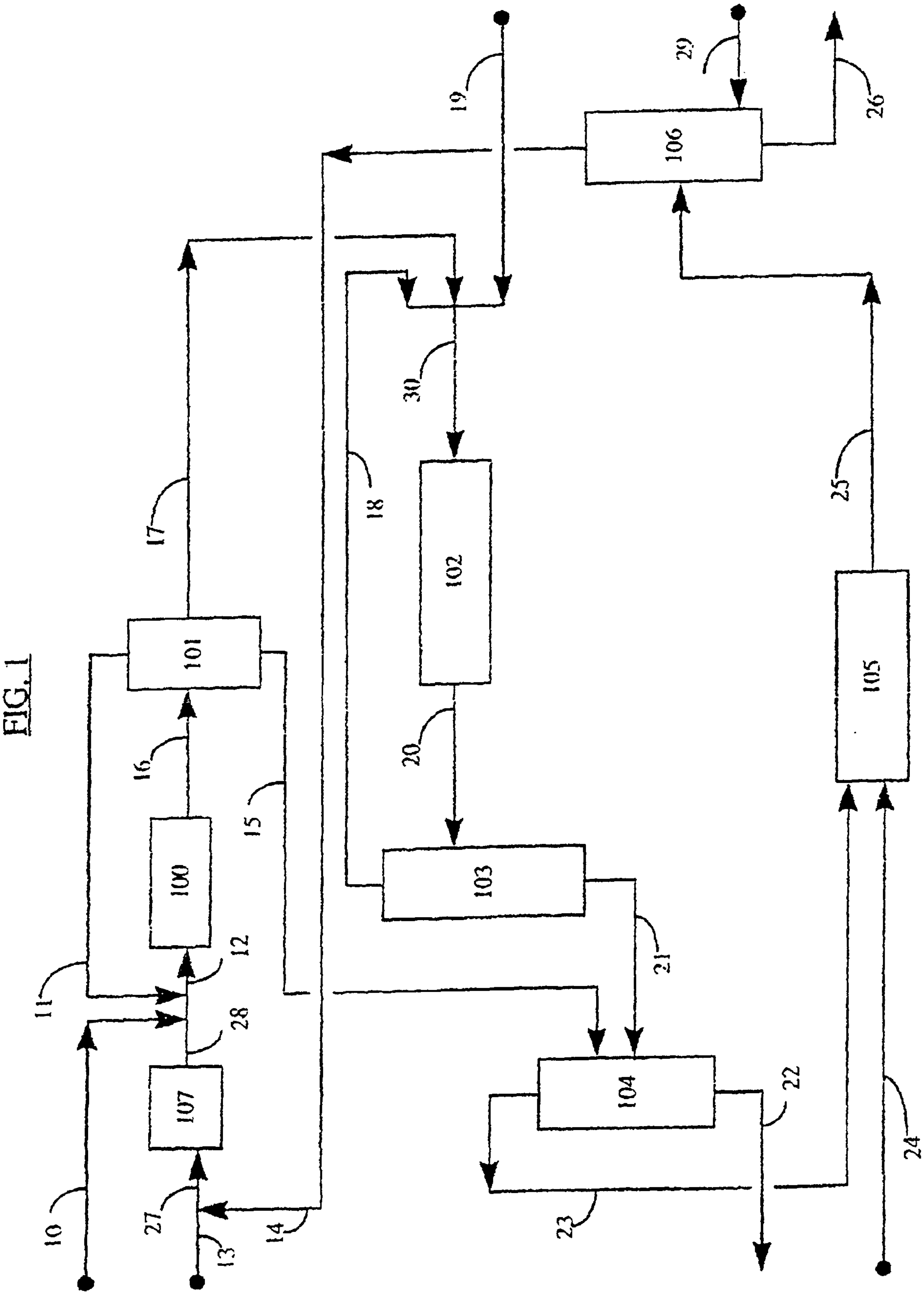
10
11
12
13
14
15
16
17
18
19
20
21
22
23
24
25
26
27
28
29
30
100
101
102
103
104
105
106
107